# EUROPEAN PATENT SPECIFICATION

(11) **EP 3 675 874 B1**
(45) Date of publication and mention of the grant of the patent: **29.11.2023**
(21) Application number: 18850513.5
(22) Date of filing: 31.08.2018
(51) Int. Cl.: A61K 31/728, A61K 38/39, A61P 17/02

(54) **PHARMACEUTICALS COMPOSITION FOR TREATING KELOID AND USES THEREOF**
PHARMAZEUTISCHE ZUSAMMENSETZUNG ZUR BEHANDLUNG VON KELOID UND VERWENDUNGEN DAVON
COMPOSITION PHARMACEUTIQUE POUR LE TRAITEMENT DES CHÉLOÏDES ET SES UTILISATIONS

(30) Priority: 01.09.2017 US 201762553267 P
(43) Date of publication of application: 08.07.2020
(73) Proprietor: Excel Med, LLC, Starkville, MS 39759 (US); National Cheng Kung University, Tainan City 701 (TW)
(72) Inventor: HUANG, Lynn, L.H., Tainan City 701 (TW)
(74) Representative: karo IP
(86) International application number: PCT/US2018/049017
(87) International publication number: WO 2019/046678

(56) References cited:
- EP-A1- 2 979 710
- EP-B1- 2 979 710
- WO-A1-2012/177257
- US-A1- 2008 206 228
- US-A1- 2009 196 927
- US-A1- 2010 283 166
- US-A1- 2014 328 826
- US-A1- 2014 328 826
- US-A1- 2014 341 866
- GERD G. GAUGLITZ ET AL: "Hypertrophic Scarring and Keloids: Pathomechanisms and Current and Emerging Treatment Strategies", MOLECULAR MEDICINE, vol. 17, no. 1-2, 5 October 2010 (2010-10-05), pages 113-125, XP055606434, Washington , DC ISSN: 1076-1551, DOI: 10.2119/molmed.2009.00153

## Description

### BACKGROUND

A keloid is a type of scar resulting from abnormal hyperplasia of skin fibrosis. The main cause is the excessive accumulation of extracellular matrix, which leads to the formation of a large scar. The scar not only has a conspicuous appearance, but also causes excessive inflammatory reaction, itching, and pain, and even leading to impaired peripheral tissue function.

Keloids occur in the high-tension positions of the human body, such as the anterior chest, suprapubic region, and upper arm. The shape of the scar is mostly crab or dumbbell shaped. Its structure, flexibility and strength are also different from normal tissues. Although a keloid scar can be removed by clinical surgery, the recurrence rate in patients is still more than 60%.

Studies have shown that the pathogenesis of keloids, in addition to family genetic factors, is mostly related to keloid fibroblasts. Compared to fibroblasts isolated from normal wound tissues, keloid fibroblasts excessively secrete extracellular matrix components, especially collagen, fibronectin, elastin, and proteoglycans, and some growth factors including vascular endothelial growth factor (VEGF), transforming growth factor (TGF), platelet-derived growth factor (PDGF), and connective tissue growth factor (CTGF). Hyperplasia of fibrous tissues in wounds of keloid patients makes the granulation tissues too large, beyond the original areas of the wounds, forming keloid scars. In addition, the expression of connexins and apoptotic genes in keloid fibroblasts is lower than normal, so the degree of apoptosis is relatively low. Also, in the scar tissue of a keloid, increase of collagen and glycosaminoglycan components causes the collagen bundles to be thickened and form a whorls arrangement. Further, the metabolic activity of the wound tissue is higher than normal tissues, resulting in hypoxia in the wound tissue, so it is easy to form a keloid under such high oxygen consumption and hypoxia expansion.

EP 2 979 710 A1 describes a cell tissue gel composition comprising hyaluronan and collagen at weight ratio 1:100 to 100:1.

GERD G. GAUGLITZ ET AL ("Hypertrophic Scarring and Keloids: Pathomechanisms and Current and Emerging Treatment Strategies", MOLECULAR MEDICINE, vol. 17, no. 1-2, 5 October 2010, pages 113-125) discloses various treatment strategies of keloids.

US 2014/328826 A1 describes the treatment of keloid scars using hyaluronan.

Current treatments of keloid mainly target keloid scars. There are many treatments including pressure dressing, local injection of steroid, laser rejuvenation, and external application of drugs. Treatments are selected according to the degree of scars such that the recurrences of keloid cannot be prevented resulting in high recurrence rates. In addition, there is currently no single treatment for all types of keloid patients, and some treatments may even cause side effects. Therefore, it is important to develop pharmaceutical compositions and treatment methods that are suitable for most keloid patients, are safe and effective, and can reduce the recurrence rates.

### SUMMARY

In one aspect, described herein is a pharmaceutical composition for use in a method of treating a keloid in a subject. The method comprises: applying a pharmaceutical composition to a keloid or an area at risk of forming a keloid in a subject, wherein the composition includes an effective amount of a hyaluronan and an effective amount of a collagen, the weight ratio per unit volume of the hyaluronan to the collagen being greater than 1.5 (e.g., 1.5-1000, 1.5-3.5, 2-5, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.5, 5, 5.5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, 900, or 1000). In some embodiments, the area at risk of forming a keloid is an area from which a keloid scar has been surgically removed.

In some embodiments, the pharmaceutical composition is produced by a procedure including: mixing a hyaluronan, a 0.001M - 0.1M phosphate buffer solution at pH 7+2, and a 0.1% - 0.9% NaCl solution to form a hyaluronan solution; providing a collagen solution; mixing the hyaluronan solution and the collagen solution at below 4°C to obtain a mixture such that the weight ratio per unit volume of hyaluronan to collagen in the mixture is greater than 1; and adjusting the pH of the mixture to 7±1. The pH of the hyaluronan solution can be below 6. In some embodiments, the pH of the collagen solution is below 6.

In some embodiments, the pharmaceutical composition further includes an additive selected from the group consisting of a nutrient, a biological active agent, an antimicrobial agent, a cell, an extracellular matrix component, and an excipient.

In some embodiments, the molecular weight of the hyaluronan is 4 to 5000 kDa (e.g., 4 to 20, 20 to 100, 100 to 500, 500 to 1000, 1000 to 2000, 2000 to 2500, 2500 to 5000, 5, 10, 50, 100, 200, 300, 400, 500, 750, 1000, 1500, 1800, 2000, 2500, 3000, 3500, 4000, 4500, or 5000 kDa).

In some embodiments, the concentration of the hyaluronan solution can be 3 to 1000 mg/ml (e.g., 3 to 200 mg/ml). In some embodiments, the concentration of the collagen solution can be 0.03 to 500 mg/ml (e.g., 1 to 100 mg/ml).

In some embodiments, the pharmaceutical composition is capable of reducing the gene expression of *plasminogen activator inhibitor-1(PAI-1*), *serpin peptidase inhibitor, clade B (Ovalbumin), plasminogen activator inhibitor-2 (PAI-2), collagen type1*, *fibronectin, alpha-smooth muscle actin (α-SMA),* or *connective tissue growth factor (CTGF)* in keloid fibroblasts.

In some embodiments, the biological active agent is selected from the group consisting of an epidermal growth factor, fibroblast growth factor (FGF), vascular endothelial growth factor (VEGF), connective tissue growth factor (CTGF), platelet-derived growth factor (PDGF), insulin-like growth factor, nerve growth factor, hepatocyte growth factor, colony-stimulating factor, stem call factor, keratinocyte growth factor, granulocyte colony-stimulating factor, granulocyte macrophage colony-stimulating factor, glial-derived neurotropic factor, ciliary neurotrophic factor, endothelial-monocyte activating polypeptide, epithelial neutrophil activating peptide, erythropoietin, bone morphogenetic protein, brain-derived neurotrophic factor, BRAK, transforming growth factor beta (TGF-β), and tumor necrosis factor.

In some embodiments, the extracellular matrix component is selected from the group consisting of a collagen, hyaluronan, gelatin, fibronectin, elastin, tenascin, laminin, vitronectin, heparan sulfate, chondroitin, chondroitin sulfate, keratin, keratan sulfate, dermatan sulfate, carrageenan, heparin, chitin, chitosan, alginate, agarose, agar, cellulose, glycogen, fibrin, fibrinogen, clotting enzymes, polyglutamic acid, and synthetic polymer or derivative thereof.

In some embodiments, the antimicrobial agent is an antibiotic, anti-microbial protein, or an anti-microbial peptide. In some embodiments, the cell is a stem cell, satellite cell, precursor cell, or tissue cell. In some embodiments, the excipient is vaseline, glycerin or lecithin. In some embodiments, the nutrient is a carbohydrate, amino acid, peptide, protein, fatty acid, lipid, vitamin, or mineral.

In another aspect, a pharmaceutical composition for treating or preventing a keloid in a subject is described herein. The composition includes an effective amount of a hyaluronan and an effective amount of a collagen, the weight ratio per unit volume of the hyaluronan to the collagen being greater than 1.5.

The details of one or more embodiments are set forth in the accompanying drawing and the description below.

### BRIEF DESCRIPTION OF DRAWINGS

FIG. 1 is a set of images that shows the morphology of normal fibroblasts and keloid fibroblasts in various pharmaceutical compositions.
FIG. 2 is a bar graph that shows the migration of normal fibroblasts and keloid fibroblasts in various pharmaceutical compositions. **P < 0.05.*
FIG. 3 is a bar graph that shows the percentages of contractile force of normal fibroblasts and keloid fibroblasts in various pharmaceutical compositions. ***P* < *0.01; *P < 0.05.*
FIG. 4A is a bar graph that shows the gene expression of *plasminogen activator inhibitor-1 (PAI-1)* in normal fibroblasts and keloid fibroblasts in various pharmaceutical compositions. ****P < 0.001; **P < 0.01; *P < 0.1.*
FIG. 4B is a bar graph that shows the gene expression of *plasminogen activator inhibitor-2 (PAI-2)* in normal fibroblasts and keloid fibroblasts in various pharmaceutical compositions. ****P < 0.001; **P < 0.01; *P < 0.1.*
FIG. 4C is a bar graph that shows the gene expression of *collagen type I* in normal fibroblasts and keloid fibroblasts in various pharmaceutical compositions. ****P < 0.001; **P < 0.01; *P< 0.1.*
FIG. 4D is a bar graph that shows the gene expression of *fibronectin* in normal fibroblasts and keloid fibroblasts in various pharmaceutical compositions. ****P < 0.001; **P < 0.01; *P< 0.1.*
FIG. 4E is a bar graph that shows the gene expression of alpha-smooth muscle actin (*α-SMA*) in normal fibroblasts and keloid fibroblasts in various pharmaceutical compositions. ****P* < *0.001; **P< 0.01; *P* < *0.1.*
FIG. 4F is a bar graph that shows the gene expression of connective tissue growth factor (*CTGF*) in normal fibroblasts and keloid fibroblasts in various pharmaceutical compositions. ****P < 0.001; **P < 0.01; *P < 0.1.*

### DETAILED DESCRIPTION

It was unexpectedly discovered that various compositions containing hyaluronan and collagen restored the morphology, migration, contractility, and expression of keloid-associated genes of keloid fibroblasts to normal levels or close to normal levels. Therefore, the compositions are used to treat keloids or reduce the risk of keloids.

### Pharmaceutical Composition

Accordingly, described herein is a pharmaceutical composition for treating keloids. The composition contains an effective amount of a hyaluronan and an effective amount of a collagen, the weight ratio per unit volume of the hyaluronan to the collagen being greater than 1.5 (e.g., 1.5-1000, 1.5-3.5, 2-5, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.5, 5, 5.5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, 900, or 1000).

The composition can further contain one or more components including a nutrient, a bioactive agent, an antimicrobial agent, a cell, an extracellular matrix component, and an excipient.

Any of the naturally-occurring collagens or their functional variants can be used for preparing the pharmaceutical composition. Collagen can be isolated and purified from collagen-rich tissues such as skin, tendon, ligament, and bone of humans and animals. Methods for isolating and purifying collagen are well known in the art. See, e.g., US Patent 5,512,291; US Patent Publication 20040138695; Methods in Enzymology, vol. 82, pp. 33-64, 1982; The Preparation of Highly Purified Insoluble Collagen, Oneson, I., et al., Am. Leather Chemists Assoc., Vol. LXV, pp. 440-450, 1970; U.S. Pat. No. 6,090,996. Collagen can also be prepared by recombinant technology, such as those described by Advanced Tissue Sciences (La Jolla, Calif.) or purchased from various venders (e.g., Fibrogen; South San Francisco, Calif.). The term "collagen" refers to the above-mentioned molecules, degraded / hydrated molecules (also named gelatin) and their derivatives. To make a collagen solution, collagen can be dissolved in acetic acid (e.g., 0.1M to 0.5M), HCl (e.g., 0.001 to 0.05 N), or water.

The term "hyaluronan" refers to a naturally-occurring anionic, non-sulfated glycosaminoglycan including repeated disaccharide units of N-acetylglucosamine and D-glucuronic acid, and its derivatives. Naturally-occurring hyaluronan (also known as hyaluronic acid or hyaluronate) can be isolated from its natural sources, e.g., capsules of *Streptococci,* rooster comb, cartilage, synovial joints fluid, umbilical cord, skin tissue and vitreous of eyes, via conventional methods. See, e.g., Guillermo Lago et al. Carbohydrate Polymers 62(4): 321-326, 2005; and Ichika Amagai et al. Fisheries Science 75(3): 805-810, 2009. Alternatively, it can be purchased from a commercial vendor, e.g., Genzyme Corporation, Lifecore Biomedical, LLC and Hyaluron Contract Manufacturing. Derivatives of naturally-occurring hyaluronan include, but are not limited to, hyaluronan esters, adipic dihydrazide -modified hyaluronan, hyaluronan amide products, crosslinked hyaluronic acid, hemiesters of succinic acid or heavy metal salts thereof hyaluronic acid, partial or total esters of hyaluronic acid, sulphated hyaluronic acid, N-sulphated hyaluronic acid, and amines or diamines modified hyaluronic acid. They can be obtained by chemically modifying one or more of its functional groups (e.g., carboxylic acid group, hydroxyl group, reducing end group, N-acetyl group). A carboxyl group can be modified via esterification or reactions mediated by carbodiimide and bishydrazide. Modifications of hydroxyl groups include, but are not limited to, sulfation, esterification, isourea coupling, cyanogen bromide activation, and periodate oxidation. A reducing end group can be modified by reductive amination. It also can be linked to a phospholipid, a dye (e.g., a fluorophore or chromophore), or an agent suitable for preparation of affinity matrices. Derivatives of naturally-occurring hyaluronan can also be obtained by crosslinking, using a crosslinking agent (e.g., bisepoxide, divinylsulfone, biscarbodiimide, small homobifunctional linker, formaldehyde, cyclohexyl isocyanide, and lysine ethyl ester, metal cation, hydrazide, or a mixture thereof) or via internal esterification, photo-crosslinking, or surface plasma treatment. The molecular weight of the hyaluronan can range from 4 kDa to 5000 kDa (e.g., 4 to 20, 20 to 100, 100 to 500, 500 to 1000, 1000 to 2000, 2000 to 2500, 2500 to 5000, 5, 10, 50, 100, 200, 300, 400, 500, 750, 1000, 1500, 1800, 2000, 2500, 3000, 3500, 4000, 4500, or 5000 kDa). To make a hyaluronan solution, hyaluronan can be dissolved in a phosphate buffer solution (e.g., 0.001~0.1 M at pH 7±2) with NaCl (e.g., 0.1~0.9%). The concentration of hyaluronan can be in the range of 3 to 1000 mg/mL. A preferred range of hyaluronan is 3 to 200 mg/mL.

The term "nutrient" refers to a source of nourishment essential for cell growth. It can be an amino acid, vitamin, mineral, carbon source (e.g., glucose), fatty acid, or a mixture thereof. In one example, the nutrient in the pharmaceutical composition is a cell growth medium, e.g., Minimum Essential Medium, Basal Medium Eagle, Dulbecco's Modified Eagle's medium, Ham's Nutrient Mixtures F-10 or F-12, Medium 199, RPMI medium, Ames' Media, BGJb Medium (Fitton-Jackson Modification), Click's Medium, CMRL-1066 Medium, Fischer's Medium, Glascow Minimum Essential Medium, Iscove's Modified Dulbecco's Medium, L-15 Medium, McCoy's 5A Modified Medium, NCTC Medium, Swim's S-77 Medium, Waymouth Medium, or William's Medium E.

A bioactive agent is any agent (e.g., peptide, polypeptide, oligosaccharide, polysaccharide, or small molecule) that improves cell viability, promotes cell proliferation, or induces cell differentiation. In one example, the bioactive agent is a growth factor, such as epidermal growth factor, fibroblast growth factor, vascular endothelial growth factor, connective tissue growth factor, platelet-derived growth factor, insulin-like growth factor, nerve growth factor, hepatocyte growth factor, colony-stimulating factors, stem cell factor, serotonin, and von Willebrand factor, transforming growth factor, keratinocyte growth factor, granulocyte colony-stimulating factor, granulocyte/macrophage colony stimulating factor, glial derived neurotrophic factor, ciliary neurotrophic factor, endothelial-monocyte activating polypeptide, epithelial neutrophil activating peptide, erythropoietin, bone morphogenetic proteins, brain-derived neurotrophic factor. In another example, the bioactive agent is a cytokine or chemokine, including, but are not limited to, IL-2, breast-expressed chemokine (e.g., BRAK), kidney-expressed chemokine (e.g., CXCL14). The bioactive agent can also be a cell differentiation factor, such as dexamethasone, sodium pyruvate, ascorbic acid-2-phosphate, retinoic acid, proline, insulin, transferrin, selenous acid, linoleic acid, and bovine serum albumin, and TGF-β3. In a preferred example, the differentiation factor is a compound that promotes chondrogenesis of mesenchymal stem cells (see those disclosed in US Patent 5,908,784), osteogenesis (e.g., dexamethasone, ascorbic acid, β-glycerol phosphate), adipogenesis (e.g., insulin, isobutyl-methyl xanthine, dexamethasone, indomethacin), cardiomyogenic differentiation (e.g., activin A, BMP-4), endothelial cell differentiation (e.g., EBM-2, dexamethasone, and VEGF), smooth muscle cell differentiation (e.g., PDGF-BB), neural induction (e.g., bFGF, EGF, and B27 supplement, DMSO, butylated hydroxyanisole, forskolin, valproic acid, KCl, K252a, and N2 supplement) and endodermal lineage differentiation(e.g., dexamethasone, HGF, and FGF-4). The bioactive agent can also be a Chinese herbal medicine or an active ingredient thereof.

Extracellular matrix components include, but are not limited to, gelatin, collagen, hyaluronan, fibronectin, elastin, tenacin, laminin, vitronectin, polypeptides, heparan sulfate, chondroitin, chondroitin sulfate, keratan, keratan sulfate, dermatan sulfate, carrageenan, heparin, chitin, chitosan, alginate, agarose, agar, cellulose, methyl cellulose, carboxyl methyl cellulose, glycogen and derivatives thereof. In addition, the extracellular matrix component can be fibrin, fibrinogen, thrombin, polyglutamic acid, a synthetic polymer (e.g., acrylate, polylactic acid, polyglycolic acid, or poly(lactic-co-glycolic acid), or a cross-linking agent (e.g., genipin, glutaraldehyde, formaldehyde, or epoxide).

One or more excipients can be included in the pharmaceutical composition to provide lubrication and moisture insulation. The presence of the excipient can also serve as a cream to bind and smooth the epithelial layer for healing of wounds. Lecithin, petroleum jelly, glycerol, glycerine, and glycerin are exemplary excipients that can be included in the cell tissue gel. Other excipients are also known and available in the art.

Cell such as stem cells, satellite cells, precursor cells, or any tissue cells can be included in the pharmaceutical composition.

The pharmaceutical composition can also include one or more antimicrobial agents, e.g., an antibiotic, anti-microbial protein, or an anti-microbial peptide.

The pharmaceutical composition can be in the form of a liquid, cream, gel, jelly, paste, ointment, spray, colloid, foam, sponge, matrix, dressing, pad, granule, suspension or powder.

To make the pharmaceutical composition, a hyaluronan solution and a collagen solution can be mixed at below 4°C to produce a mixture that contain hyaluronan and collagen at the desired weight ratio per unit volume. A preferred weight ratio of hyaluronan to collagen ranges from 1.5 to 1000. One or more other components (e.g., nutrients, bioactive agents, antimicrobial agents, cells, extracellular matrix components, and excipients) can be added to the mixture. The pH of the final mixture is then adjusted to neutral or near neutral (e.g., 7±1) to form the pharmaceutical composition, which has a gelatinous consistency. The concentration of the hyaluronan solution can be 3 to 1000 mg/ml (e.g., 3 to 100 mg/ml, 5 mg/ml to 10 mg/ml, 7 mg/ml to 15 mg/ml, 10 to 25 mg/ml, 15 to 35 mg/ml, 25 to 50 mg/ml, 35 to 65 mg/ml, 50 to 75 mg/ml, 65 to 85 mg/ml, 75 to 95 mg/ml, 85 to 105 mg/ml, 100 to 400 mg/ml, 250 to 550 mg/ml, 400 to 700 mg/ml, 550 to 850 mg/ml, 700 to 1000 mg/ml, 0.5 mg/ml, 1 mg/ml, 1.5 mg/ml, 3 mg/ml, 5 mg/ml, 10 mg/ml, 15 mg/ml, 20 mg/ml, 25 mg/ml, 30 mg/ml, 35 mg/ml, 45 mg/ml, 50 mg/ml, 55 mg/ml, 60 mg/ml, 65 mg/ml, 70 mg/ml, 75 mg/ml, 80 mg/ml, 85 mg/ml, 90 mg/ml, 95 mg/ml, 100 mg/ml, 200 mg/ml, 300 mg/ml, 400 mg/ml, 500 mg/ml, 600 mg/ml, 700 mg/ml, 800 mg/ml, 900 mg/ml, or 1000 mg/ml). A preferred range for the hyaluronan is 3 to 200 mg/ml. The concentration of the collagen solution can be 0.03 to 500 mg/ml (e.g., 0.03 to 1 mg/ml, 1 to 2.5 mg/ml, 2.5 to 5 mg/ml, 5 mg/ml to 10 mg/ml, 7 mg/ml to 15 mg/ml, 10 to 25 mg/ml, 15 to 35 mg/ml, 25 to 50 mg/ml, 35 to 65 mg/ml, 50 to 75 mg/ml, 65 to 85 mg/ml, 75 to 95 mg/ml, 85 to 105 mg/ml, 100 to 200 mg/ml, 150 to 250 mg/ml, 200 to 300 mg/ml, 250 to 350 mg/ml, 300 to 400 mg/ml, 350 to 450 mg/ml, 400 to 500 mg/ml, 0.5 mg/ml, 1 mg/ml, 1.5 mg/ml, 3 mg/ml, 5 mg/ml, 10 mg/ml, 15 mg/ml, 20 mg/ml, 25 mg/ml, 30 mg/ml, 35 mg/ml, 45 mg/ml, 50 mg/ml, 55 mg/ml, 60 mg/ml, 65 mg/ml, 70 mg/ml, 75 mg/ml, 80 mg/ml, 85 mg/ml, 90 mg/ml, 95 mg/ml, 100 mg/ml, 200 mg/ml, 300 mg/ml, 400 mg/ml, or 500 mg/ml). A preferred range for the collagen is 1 to 100 mg/ml.

### Treatment Method

The pharmaceutical composition can be administered to a patient to treat, prevent, or reduce the reoccurrence of a keloid. The composition can be injected to or applied topically over a keloid or an area at risk of developing a keloid. In particular, the composition can be administered to an area from which a keloid has been removed surgically to aid wound healing and reduce the risk of a keloid forming at the same area.

The composition can be applied to a subject as often as needed, e.g., 1 to 5 times daily, 1 to 5 times per week, 1 to 5 times per month, for a suitable treatment period, e.g., 1 to 4 week, 1 to 12 months, or 1 to 3 years.

"Treating" refers to administration of a composition to a subject, who is suffering from or is at risk for developing a disorder, with the purpose to cure, alleviate, relieve, remedy, delay the onset of, prevent, or ameliorate the disorder, the symptom of the disorder, the disease state secondary to the disorder, or the predisposition toward the disorder. An "effective amount" refers to an amount of the composition that is capable of producing a medically desirable result in a treated subject. The treatment method can be performed alone or in conjunction with other drugs or therapies.

The specific examples below are to be construed as merely illustrative, and not limitative of the remainder of the disclosure in any way whatsoever. Without further elaboration, it is believed that one skilled in the art can, based on the description herein, utilize the present disclosure to its fullest extent.

### Example 1: Preparation of pharmaceutical compositions

A hyaluronan solution was prepared by mixing hyaluronan, a phosphate buffer solution (0.001 to 0.1 M and pH 7±2), and a NaCl solution (0.1 to 0.9 %). A collagen solution was mixed with the hyaluronan solution in each of the centrifuge tubes in the weight ratios shown in Tables 1, 2, and 3. A mixture containing fetal bovine serum and a cell culture medium was added to each tube so that the final volume in each tube was the same. Subsequently, the pH of each tube was measured with a pH acid-base test paper, and the pH was adjusted to neutral (pH 7.0) with HCl and NaOH. The mixing steps were carried out at below 4°C.

**Table 1: Weight ratio and ratio of hyaluronan to collagen per unit volume**

| **Groups** | Weight ratio of hyaluronan to collagen | hyaluronan : collagen final conc. (mg/mL) | Molecular weights of hyaluronan |
|---|---|---|---|
| Control Group 1 | - | 0 : 3 | |
| Experimental Group 1 | 1 | 3 : 3 | 1~1.8 MDa |
| Experimental Group 2 | 2 | 6 : 3 | 1~1.8 MDa |
| Experimental Group 3 | 3 | 9 : 3 | 1~1.8 MDa |

**Table 2: Weight ratio and ratio of hyaluronan to collagen per unit volume**

| **Groups** | Weight ratio of hyaluronan to collagen | hyaluronan : collagen final conc. (mg/mL) | Molecular weights of hyaluronan |
|---|---|---|---|
| Control Group 2 | - | 0 : 10 | |
| Experimental Group 4 | 1 | 10 : 10 | 1~1.8 MDa |
| Experimental Group 5 | 1.5 | 15 : 10 | 1~1.8 MDa |
| Experimental Group 6 | 2 | 20 : 10 | 1~1.8 MDa |
| Experimental Group 7 | 2 | 20 : 10 | 4~20 KDa |
| Experimental Group 8 | 2 | 20 : 10 | 21~99 KDa |
| Experimental Group 9 | 2 | 20 : 10 | 0.1~1 MDa |
| Experimental Group 10 | 2 | 20 : 10 | 1.8~5 MDa |

**Table 3: Weight ratio and ratio of hyaluronan to collagen per unit volume**

| **Groups** | Weight ratio of hyaluronan to collagen | hyaluronan : collagen final conc. (mg/mL) | Molecular weights of hyaluronan |
|---|---|---|---|
| Control Group 3 | - | 0 : 50 | |
| Experimental Group 11 | 1 | 50 : 50 | 4~20 KDa |
| Experimental Group 12 | 1.5 | 150 : 100* | 0.1~1 MDa |
| Experimental Group 13 | 2 | 1000 : 500* | 4~20 KDa |
| Experimental Group 14 | 5 | 25 : 5 | 1~1.8 MDa |
| Experimental Group 15 | 10 | 200 : 20 | 21~99 KDa |
| Experimental Group 16 | 50 | 50 : 1 | 0.1~1 MDa |
| Experimental Group 17 | 100 | 3 : 0.03 | 1~1.8 MDa |
| Experimental Group 18 | 1000 | 100 : 0.1 | 21~99 KDa |

| | | | |
|---|---|---|---|
| *: hydrated collagen was used. Control groups 1-3 and Experimental groups 1,4,11 do not represent the invention. | | | |

### Example 2: Morphologies

Previous studies suggested that keloid fibroblasts exhibit fusiform attachment under normal culturing conditions. In order to understand the effect of various compositions containing hyaluronan and collagen on the morphology of keloid fibroblasts, normal fibroblasts (NF) and keloid fibroblasts (KF) were cultured in the control groups and experimental groups of Tables 1-3, and in a culture medium (10 mL DMEM, 10% FBS, 1% penicillin/streptomycin). The fibroblasts were stained with 0.5% crystal violet and observed under an electron microscope.

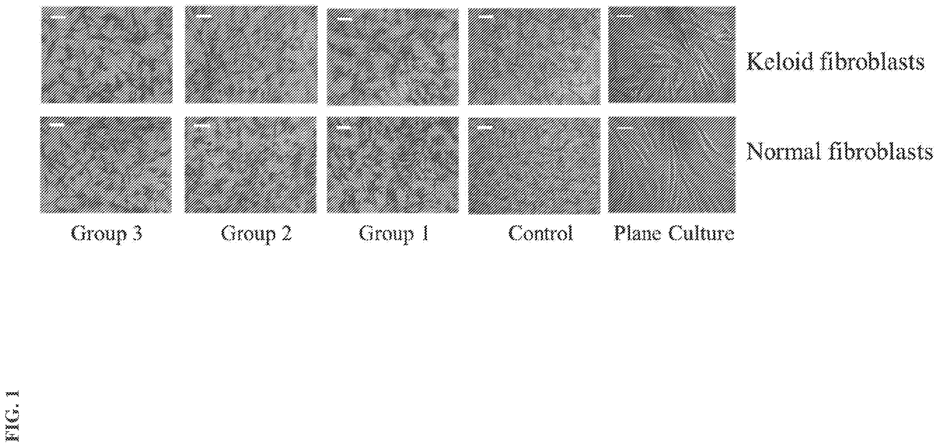

As shown in Fig. 1, in the culture medium (plane culture group), the appearances of the normal fibroblasts and keloid fibroblasts were not significantly different, and both attached in fusiform. Both proliferated in a similar arrangement, but the density of the keloid fibroblasts after culturing was significantly greater than that of the normal fibroblasts. In the collagen-only composition (control group), the normal fibroblasts exhibited significantly more filopodia than that of the plane culture group and grew outward. The protrusions between cells were connected to each other to form an interlaced network. Similar to the normal fibroblasts, the keloid fibroblasts also exhibited filopodia and formed an interlaced network. On the other hand, in experimental group 1, the keloid fibroblasts were more hypertrophic near the nucleus, and were more protruded than normal fibroblasts. However, in experimental groups 2 and 3, the protuberances of both normal fibroblasts and keloid fibroblasts became reduced. Their overall morphology and growth were similar in experimental groups 2, 3, 5 to 10, and 12 to 18. The data showed that keloid fibroblasts cultured in the compositions containing hyaluronan and collagen were able to return to a normal morphology.

### Example 3: Migration test

Previous studies have shown that keloid fibroblasts have higher migration potential than normal fibroblasts. A study was carried out in order to understand the effect of various compositions containing hyaluronan and collagen on the migration of keloid fibroblasts. 100 µL of each composition of the groups was apply to coating on a culture dish and an O-ring (inner diameter 4 mm, outer diameter 6 mm) was placed in the center of the culture dish. Normal fibroblasts or keloid fibroblasts were placed inside the O-ring respectively in each coated dish. The O-ring was removed after the cells attached, and the cells continued to culture for four days. The cells were than stained with crystal violet and observed. Cell migration areas were calculated.

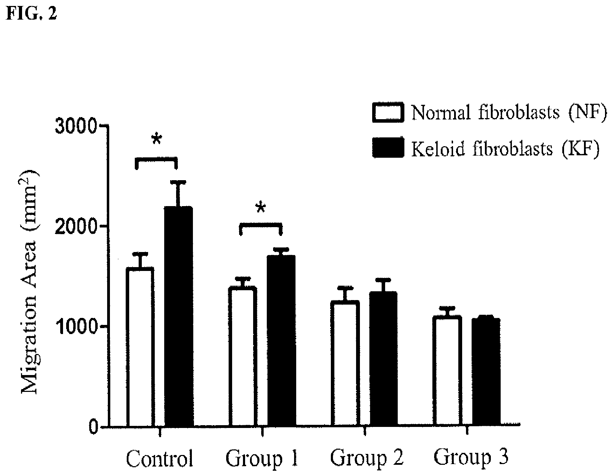

As shown in Fig. 2, in the collagen-only group (control group), the migration area of the keloid fibroblasts was significantly larger than that of the normal fibroblasts. However, in the experimental groups 2 and 3, the migration area of the keloid fibroblasts was not significantly different from that of the normal fibroblasts. Similar results were observed in the experimental groups 5 to 10 and 12 to 18. The results suggest that compositions containing hyaluronan and collagen with the weight ratio greater than 1 have the ability to restore the migration ability of keloid fibroblasts to a normal level.

### Example 4: Contraction test

Previous studies have shown that keloid fibroblasts have stronger contractile force than normal fibroblasts. A study was carried out in order to understand the effect of various compositions containing hyaluronan and collagen on the contractile force of keloid fibroblasts.

Normal or keloid fibroblasts were each added to the various compositions containing hyaluronan and collagen. After mixing uniformly, 50 µL of each was dropped onto the center of a culture dish in a half-spherical shape, which was placed at 37°C in an incubator for 2 hours. After 2 hours of gelation, the area (A₀) of the half-sphere was recorded. After 800 µL of culture medium was added, the fibroblasts started to contract the composition and the half-sphere was shrunken. After 48 hours, the area (A) of the half-sphere was recorded. The following formula was used to calculate the percentage of cell contractile force: X = [(A₀-A)/A_{0]} ×100%. A higher percentage of shrinkage indicated a stronger contractile force.

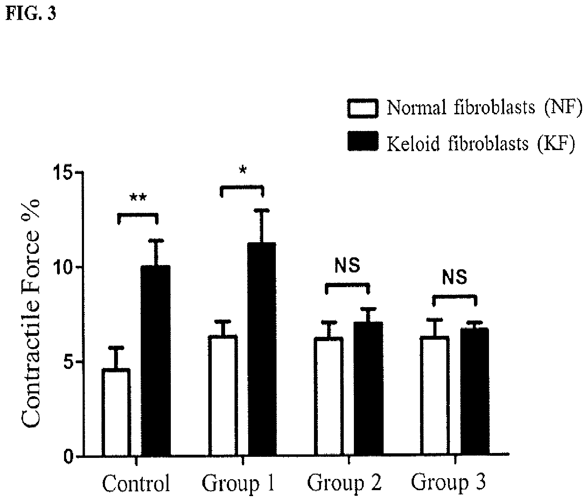

As shown in Fig. 3, the percentage of contractile force of the keloid fibroblasts was significantly higher than that of the normal fibroblasts in the control group and experimental group 1. However, there was no significant difference between the percentages of contractile force of the keloid fibroblasts and the normal fibroblasts in the experimental groups 2 and 3. Similar results were observed in the experimental groups 5 to 10 and 12 to18. The results suggest that compositions containing hyaluronan and collagen with a weight ratio greater than 1 have the ability to reduce the contractile ability of keloid fibroblast to a normal level.

### Example 5: Gene expression analysis

Previous studies have demonstrated that tension factors (e.g., PAI-1, PAI-2 and α-SMA), growth factor CTGF, and extracellular matrix components (collagen I and fibronectin) are associated with the formation of keloid. A study was carried out in order to understand the effect of various compositions containing hyaluronan and collagen on keloid fibroblasts on the molecular level.

Normal and keloid fibroblasts were separately cultured in the plane culture group, control group, and experimental groups described in Table 1. After four days of culturing, total RNA was extracted from the fibroblasts. The total RNA was then reverse transcribed into cDNA and analyzed by quantitative polymerase chain reaction (PCR). In the plane culture group, cells were cultured on the surface of a standard culture dish.

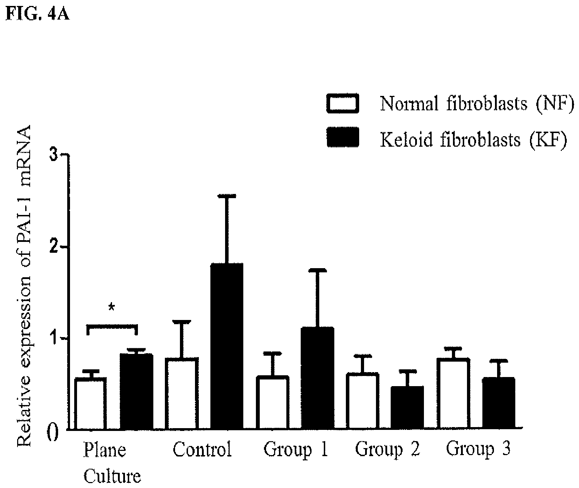

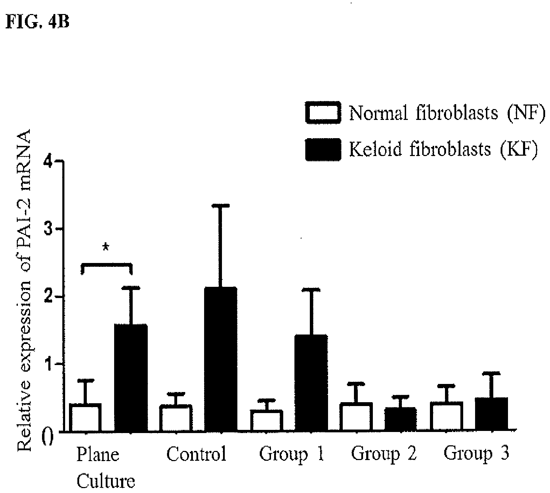

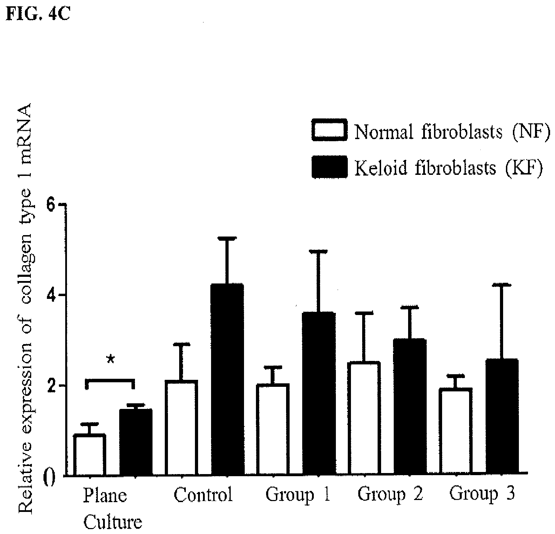

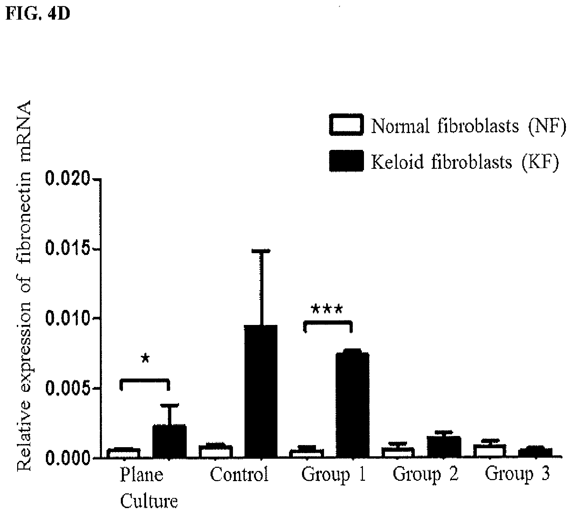

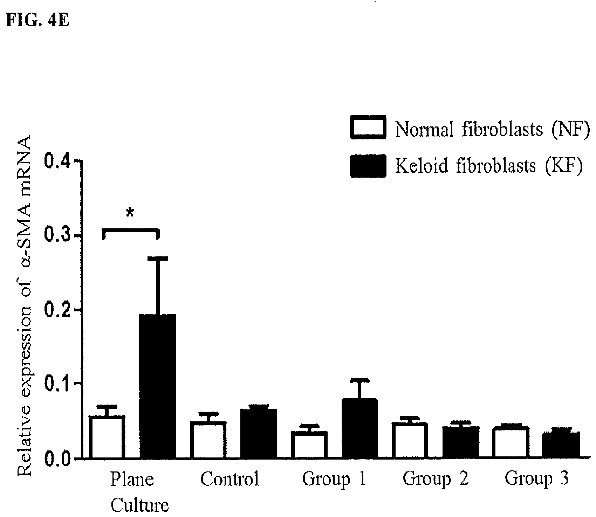

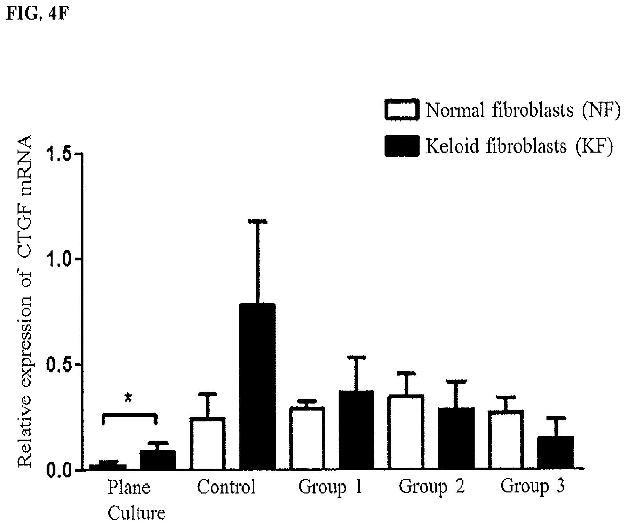

As shown in Figs 4A-4F, in the plane culture group, the control group, and the experimental group 1, the expression levels of *PAI-1, PAI-2, collagen I, fibronectin, α-SMA,* and *CTGF* in the keloid fibroblasts were higher than those in the normal fibroblasts. On the other hand, in the experimental groups 2 and 3, the expression levels of these genes in keloid fibroblasts decreased and were not significantly different from those in the normal fibroblasts. The results suggest that compositions containing hyaluronan and collagen with a weight ratio greater than 1 can decrease the expression levels of genes associated with keloid formation.

In summary, compositions containing hyaluronan and collagen with a weight ratio greater than 1 were shown to improve the morphology, migration, and contractility of keloid fibroblasts to normal levels. The expression levels of keloid-associated genes were also modulated to be close to those in normal fibroblasts by the compositions. Therefore, the compositions can reduce hyperplasia of healing wound tissues in keloid patients such that the skin is closer to normal skin without abnormal protrusions. Furthermore, the compositions can be used to treat patients after surgical removal of keloid scars to prevent hyperplasia of the healing tissues.

## Claims

1. A pharmaceutical composition for use in the treatment or prevention of keloid, wherein the composition includes an effective amount of a hyaluronan and an effective amount of a collagen, the weight ratio of the hyaluronan to the collagen is equal to or greater than 1.5.

2. The pharmaceutical composition of claim 1, wherein the weight ratio of the hyaluronan to the collagen is equal to or greater than 2.

3. The pharmaceutical composition of claim 2, wherein the weight ratio of the hyaluronan to the collagen is equal to or greater than 3.

4. The pharmaceutical composition of any of claims 1-3, wherein the pharmaceutical composition is produced by a procedure including:
mixing a hyaluronan, a 0.001M to 0.1M phosphate buffer solution at pH 7±2, and a 0.1% to 0.9% NaCl solution to form a hyaluronan solution;
providing a collagen solution;
mixing the hyaluronan solution and the collagen solution at below 4°C to obtain a mixture such that the weight ratio per unit volume of hyaluronan to collagen in the mixture is greater than 1; and
adjusting the pH of the mixture to 7±1.

5. The pharmaceutical composition of claim 4, wherein the pH of the hyaluronan solution is below 6.

6. The pharmaceutical composition of claim 4, wherein the pH of the collagen solution is below 6.

7. The pharmaceutical composition of any of claims 1-3, wherein the pharmaceutical composition further includes an additive selected from the group consisting of a nutrient, a biological active agent, an antimicrobial agent, a cell, an extracellular matrix component, and an excipient.

8. The pharmaceutical composition of any of claims 1-3, wherein the molecular weight of the hyaluronan is 4 to 5000 kDa.

9. The pharmaceutical composition of any of claims 1-3, wherein the pharmaceutical composition is capable of reducing the gene expression of plasminogen activator inhibitor-1(PAI-1), serpin peptidase inhibitor, clade B (Ovalbumin), plasminogen activator inhibitor-2 (PAI-2), collagen type1, fibronectin, alpha-smooth muscle actin (α-SMA), or connective tissue growth factor (CTGF) in keloid fibroblasts.

10. The pharmaceutical composition of claim 7, wherein the biological active agent is selected from the group consisting of an epidermal growth factor, fibroblast growth factor (FGF), vascular endothelial growth factor (VEGF), connective tissue growth factor (CTGF), platelet-derived growth factor (PDGF), insulin-like growth factor, nerve growth factor, hepatocyte growth factor, colony-stimulating factor, stem call factor, keratinocyte growth factor, granulocyte colony-stimulating factor, granulocyte macrophage colony-stimulating factor, glial-derived neurotropic factor, ciliary neurotrophic factor, endothelial-monocyte activating polypeptide, epithelial neutrophil activating peptide, erythropoietin, bone morphogenetic protein, brain-derived neurotrophic factor, BRAK, transforming growth factor beta (TGF-β), and tumor necrosis factor.

11. The pharmaceutical composition of claim 7, wherein the extracellular matrix component is selected from the group consisting of a collagen, hyaluronan, gelatin, fibronectin, elastin, tenascin, laminin, vitronectin, heparan sulfate, chondroitin, chondroitin sulfate, keratin, keratan sulfate, dermatan sulfate, carrageenan, heparin, chitin, chitosan, alginate, agarose, agar, cellulose, glycogen, fibrin, fibrinogen, clotting enzymes, polyglutamic acid, and synthetic polymer or derivative thereof.

12. The pharmaceutical composition of claim 7, wherein antimicrobial agent is an antibiotic, anti-microbial protein, or an anti-microbial peptide.

13. The pharmaceutical composition of claim 7, wherein the cell is a stem cell, satellite cell, precursor cell, or tissue cell.

14. The pharmaceutical composition of claim 7, wherein the excipient is vaseline, glycerin or lecithin.

15. The pharmaceutical composition of claim 7, wherein the nutrient is a carbohydrate, amino acid, peptide, protein, fatty acid, lipid, vitamin, or mineral.

## Patentansprüche

1. Eine pharmazeutische Zusammensetzung zur Verwendung bei der Behandlung oder Vorbeugung von Keloiden, wobei die Zusammensetzung eine wirksame Menge eines Hyaluronans und eine wirksame Menge eines Kollagens umfasst, wobei das Gewichtsverhältnis von Hyaluronan zu Kollagen gleich oder größer als 1,5 ist.

2. Pharmazeutische Zusammensetzung nach Anspruch 1, wobei das Gewichtsverhältnis von Hyaluronan zu Kollagen gleich oder größer als 2 ist.

3. Pharmazeutische Zusammensetzung nach Anspruch 2, wobei das Gewichtsverhältnis von Hyaluronan zu Kollagen gleich oder größer als 3 ist.

4. Pharmazeutische Zusammensetzung nach einem der Ansprüche 1 bis 3, wobei die pharmazeutische Zusammensetzung durch ein Verfahren hergestellt wird, das Folgendes umfasst:
Mischen eines Hyaluronans, einer 0,001M bis 0,1M Phosphatpufferlösung bei pH 7±2 und einer 0,1% bis 0,9%igen NaCl-Lösung, um eine Hyaluronanlösung herzustellen;
Bereitstellen einer Kollagenlösung;
Mischen der Hyaluronanlösung und der Kollagenlösung bei unter 4°C, um eine Mischung zu erhalten, bei der das Gewichtsverhältnis pro Volumeneinheit von Hyaluronan zu Kollagen in der Mischung größer als 1 ist; und
Einstellen des pH-Werts der Mischung auf 7±1.

5. Pharmazeutische Zusammensetzung nach Anspruch 4, wobei der pH-Wert der Hyaluronanlösung unter 6 liegt.

6. Pharmazeutische Zusammensetzung nach Anspruch 4, wobei der pH-Wert der Kollagenlösung unter 6 liegt.

7. Pharmazeutische Zusammensetzung nach einem der Ansprüche 1 bis 3, wobei die pharmazeutische Zusammensetzung ferner einen Zusatzstoff enthält, ausgewählt aus der Gruppe bestehend aus: Ein Nährstoff, ein biologischer Wirkstoff, ein antimikrobielles Mittel, einer Zelle, einer extrazellulären Matrixkomponente und einem Hilfsstoff.

8. Pharmazeutische Zusammensetzung nach einem der Ansprüche 1-3, wobei das Molekulargewicht des Hyaluronans 4 bis 5000 kDa beträgt.

9. Pharmazeutische Zusammensetzung nach einem der Ansprüche 1 bis 3, wobei die pharmazeutische Zusammensetzung in der Lage ist, die Genexpression von PlasminogenAktivator-Inhibitor-1 (PAI-1), Serpin-Peptidase-Inhibitor, Klade B (Ovalbumin), PlasminogenAktivator-Inhibitor-2 (PAI-2), Kollagen Typ 1, Fibronektin, Alpha-Glattmuskel-Actin (α-SMA) oder Bindegewebs-Wachstumsfaktor (CTGF) in Keloid-Fibroblasten zu reduzieren.

10. Pharmazeutische Zusammensetzung nach Anspruch 7, wobei der biologische Wirkstoff ausgewählt ist aus der Gruppe bestehend aus: Epidermaler Wachstumsfaktor, Fibroblasten-Wachstumsfaktor (FGF), vaskulärer endothelialer Wachstumsfaktor (VEGF), Bindegewebs-Wachstumsfaktor (CTGF), von Blutplättchen abgeleiteter Wachstumsfaktor (PDGF), insulinähnlicher Wachstumsfaktor, Nerven-Wachstumsfaktor, Hepatozyten-Wachstumsfaktor, koloniestimulierender Faktor, Stammzellfaktor, Keratinozyten-Wachstumsfaktor, Granulozyten-Kolonie-stimulierender Faktor, Granulozyten-Makrophagen-Kolonie-stimulierender Faktor, glial abgeleiteter neurotropischer Faktor, ziliärer neurotropher Faktor, Endothel-Monozyten aktivierendes Polypeptid, epitheliales Neutrophilenaktivierendes Peptid, Erythropoietin, Knochen-morphogenetisches Protein, Gehirn-abgeleiteter neurotropher Faktor, BRAK, transformierender Wachstumsfaktor beta (TGF-β), und Tumor-Nekrose-Faktor.

11. Pharmazeutische Zusammensetzung nach Anspruch 7, wobei die extrazelluläre Matrixkomponente ausgewählt ist aus der Gruppe bestehend aus: Kollagen, Hyaluronan, Gelatine, Fibronectin, Elastin, Tenascin, Laminin, Vitronectin, Heparansulfat, Chondroitin, Chondroitinsulfat, Keratin, Keratansulfat, Dermatansulfat, Carrageenan, Heparin, Chitin, Chitosan, Alginat, Agarose, Agar, Cellulose, Glykogen, Fibrin, Fibrinogen, Gerinnungsenzyme, Polyglutaminsäure und ein synthetisches Polymer oder Derivat davon.

12. Pharmazeutische Zusammensetzung nach Anspruch 7, wobei das antimikrobielle Mittel ein Antibiotikum, ein antimikrobielles Protein oder ein antimikrobielles Peptid ist.

13. Pharmazeutische Zusammensetzung nach Anspruch 7, wobei die Zelle eine Stammzelle, Satellitenzelle, Vorläuferzelle oder Gewebezelle ist.

14. Pharmazeutische Zusammensetzung nach Anspruch 7, wobei der Hilfsstoff Vaseline, Glycerin oder Lecithin ist.

15. Pharmazeutische Zusammensetzung nach Anspruch 7, wobei der Nährstoff ein Kohlenhydrat, eine Aminosäure, ein Peptid, ein Protein, eine Fettsäure, ein Lipid, ein Vitamin oder ein Mineral ist.

## Revendications

1. Composition pharmaceutique pour une utilisation dans le traitement ou la prévention de chéloïdes, la composition comprenant une quantité efficace d'un hyaluronane et une quantité efficace d'un collagène, le rapport en poids de l'hyaluronane sur le collagène étant égal ou supérieur à 1,5.

2. Composition pharmaceutique selon la revendication 1, le rapport en poids de l'hyaluronane sur le collagène étant égal ou supérieur à 2.

3. Composition pharmaceutique selon la revendication 2, le rapport en poids de l'hyaluronane sur le collagène étant égal ou supérieur à 3.

4. Composition pharmaceutique selon l'une quelconque des revendications 1 à 3, la composition pharmaceutique étant produite par une procédure comprenant
le mélange d'un hyaluronane, d'une solution de tampon phosphate de 0,001 M à 0,1 M à pH 7 ± 2 et d'une solution de NaCl à 0,1 % à 0,9 % pour former une solution de hyaluronane ;
la fourniture d'une solution de collagène ;
le mélange de la solution de hyaluronane et de la solution de collagène à une température inférieure à 4 °C pour obtenir un mélange tel que le rapport en poids par unité de volume de hyaluronane sur collagène dans le mélange soit supérieur à 1 ; et
l'ajustement du pH du mélange à 7 ± 1.

5. Composition pharmaceutique selon la revendication 4, le pH de la solution de hyaluronane étant inférieur à 6.

6. Composition pharmaceutique selon la revendication 4, le pH de la solution de collagène étant inférieur à 6.

7. Composition pharmaceutique selon l'une quelconque des revendications 1 à 3, la composition pharmaceutique comprenant en outre un additif choisi dans le groupe constitué par un nutriment, un agent actif biologique, un agent antimicrobien, une cellule, un composant de matrice extracellulaire et un excipient.

8. Composition pharmaceutique selon l'une quelconque des revendications 1 à 3, le poids moléculaire du hyaluronane étant de 4 à 5 000 kDa.

9. Composition pharmaceutique selon l'une quelconque des revendications 1 à 3, la composition pharmaceutique étant capable de réduire l'expression génique de l'inhibiteur 1 de l'activateur du plasminogène (PAI-1), de l'inhibiteur de la serpine peptidase, du clade B (ovalbumine), de l'inhibiteur 2 de l'activateur du plasminogène (PAI-2), du collagène de type 1, de la fibronectine, de l'actine alpha de muscle lisse (α-SMA) ou du facteur de croissance de tissus connectifs (CTGF) dans des fibroblastes chéloïdes.

10. Composition pharmaceutique selon la revendication 7, l'agent actif biologique étant choisi dans le groupe constitué par un facteur de croissance épidermique, un facteur de croissance des fibroblastes (FGF), un facteur de croissance endothéliale vasculaire (VEGF), un facteur de croissance de tissus connectifs (CTGF), un facteur de croissance issu de plaquettes (PDGF), un facteur de croissance similaire à l'insuline, un facteur de croissance de nerfs, un facteur de croissance d'hépatocytes, un facteur de stimulation de colonie, un facteur de cellules souches, un facteur de croissance de kératinocytes, un facteur de stimulation de colonie de granulocytes, un facteur de stimulation de colonie de macrophages de granulocytes, un facteur neurotropique issu des gliales, un facteur neurotrophique ciliaire, un polypeptide activant les monocytes endothéliaux, un peptide activant les neutrophiles épithéliaux, l'érythropoïétine, une protéine morphogénétique osseuse, un facteur neurotrophique issu du cerveau, BRAK, un facteur de croissance de transformation bêta (TGF-β) et un facteur de nécrose tumorale.

11. Composition pharmaceutique selon la revendication 7, le composant de matrice extracellulaire étant choisi dans le groupe constitué par un collagène, un hyaluronane, une gélatine, une fibronectine, une élastine, une ténascine, une laminine, une vitronectine, un sulfate d'héparane, une chondroïtine, un sulfate de chondroïtine, une kératine, un sulfate de kératane, un sulfate de dermatane, un carraghénane, une héparine, une chitine, un chitosane, un alginate, un agarose, un agar, une cellulose, un glycogène, une fibrine, un fibrinogène, des enzymes de coagulation, un poly(acide glutamique) et un polymère synthétique ou un dérivé correspondant.

12. Composition pharmaceutique selon la revendication 7, un agent antimicrobien étant un antibiotique, une protéine antimicrobienne ou un peptide antimicrobien.

13. Composition pharmaceutique selon la revendication 7, la cellule étant une cellule souche, une cellule satellite, une cellule précurseur ou une cellule de tissu.

14. Composition pharmaceutique selon la revendication 7, l'excipient étant la vaseline, la glycérine ou la lécithine.

15. Composition pharmaceutique selon la revendication 7, le nutriment étant un glucide, un acide aminé, un peptide, une protéine, un acide gras, un lipide, une vitamine ou un minéral.
